# EUROPÄISCHE PATENTSCHRIFT

(11) **EP 2 742 916 B1**
(45) Veröffentlichungstag und Bekanntmachung des Hinweises auf die Patenterteilung: **06.12.2017**
(21) Anmeldenummer: 13197735.7
(22) Anmeldetag: 17.12.2013
(51) Int. Cl.: A61F 2/91, A61F 2/01, B23K 26/364, B23K 26/362

(54) **Medizinisches Implantat, Behandlungssystem mit einem derartigen Implantat und Verfahren zum Herstellen eines Implantats**
Medical implant, treatment system with such an implant and method for producing an implant
Implant médical, système de traitement comprenant un tel implant et procédé de fabrication d'un implant

(30) Priorität: 17.12.2012 DE 102012112366; 29.11.2013 DE 102013113271
(43) Veröffentlichungstag der Anmeldung: 18.06.2014
(73) Patentinhaber: Acandis GmbH & Co. KG, 76327 Pfinztal (DE)
(72) Erfinder: Nagl, Frank, 76137 Karlsruhe (DE)
(74) Vertreter: Kilchert, Jochen

(56) Entgegenhaltungen:
- WO-A1-2013/178297
- DE-A1- 19 722 429
- US-A1- 2002 065 553
- ROHDE H ET AL: "PRAEZISIONSGEOMETRIEN FEINSTSCHNEIDANWENDUNGEN MIT DEM LASER", LASER PRAXIS, CARL HANSER VERLAG, MUENCHEN, DE, Nr. 3, 1. November 2000 (2000-11-01), Seite 28,30, XP001008935, ISSN: 0937-7069

## Beschreibung

Die Erfindung betrifft ein medizinisches Implantat, ein Behandlungssystem mit einem derartigen Implantat sowie ein Verfahren zum Herstellen eines medizinischen Implantats.

Medizinische Implantate mit einer radial komprimierbaren und radial expandierbaren Umfangswandung, beispielsweise Stents, werden meist aus einem Geflecht aus Drähten oder einer Zellenstruktur gebildet, wobei die Zellen durch Stege begrenzt sind. Die Stege sind einstückig miteinander verbunden und bilden so die Umfangswandung, deren Querschnittsdurchmesser variabel ist. Die Herstellung von aus Stegen gebildeten Umfangswandungen erfolgt durch einen schneidenden Prozess, wobei ein Röhrchen bereitgestellt wird, aus dessen Wandung die Zellen der herzustellenden Umfangswandung ausgeschnitten werden. Durch das Ausschneiden der Zellen werden die Stege gebildet, die eine rechteckige oder trapezförmige Querschnittskontur aufweisen.

Die trapezförmige Querschnittskontur der Stege begrenzt die Komprimierbarkeit. Der kleinstmögliche komprimierte Querschnittsdurchmesser entspricht im Wesentlichen dem Querschnittsdurchmesser des Röhrchens, das die Vorlage für den Stent bildet. Das Röhrchen muss jedoch einen Mindestdurchmesser aufweisen, um einerseits die gewünschte Wandstärke für den Stent bereitzustellen und andererseits einen Durchgangskanal offen zu lassen, der das Röhrchen in Längsrichtung durchdringt.

Bei der Herstellung eines Implantats aus einem Röhrchen ergibt sich die Schwierigkeit, dass das Röhrchen wegen der meist gewünschten geringen Wandstärke, vergleichsweise instabil ist. Dies stellt hohe Anforderungen an eine Halterung bzw. Lagerung dar. Das entsprechende Werkzeug zur schneidenden Bearbeitung des Röhrchens ist daher so auszulegen, dass die Röhrchenform beim Greifen des Röhrchens nicht verändert, gleichzeitig aber das Röhrchen ausreichend fest gehalten wird, um die Zellen aus der Wandung des Röhrchens auszuschneiden.

DE 197 22 429 A1 beschreibt eine Vorrichtung zum Entfernen von Gallensteinen. Die Vorrichtung umfasst ein expandierbares Fangelement, das durch Längsschnitte in einem Draht gebildet ist, wobei der Draht im Gebrauch aus dem Körper eines Patienten führt, um ein Herausziehen des Fangelements zu ermöglichen. Nachteilig ist bei der bekannten Vorrichtung, dass die Bewegung und Anordnung des Fangelements innerhalb des Körpers des Patienten wegen eingeschränkter röntgensichtbarer Eigenschaften nur schwer beobachtet und kontrolliert werden kann. Außerdem eignet sich die Vorrichtung nicht zum Verbleib innerhalb des Körpers und ist daher nicht als Implantat einsetzbar.

Der Erfindung liegt die Aufgabe zugrunde, ein medizinisches Implantat anzugeben, das sich einfach herstellen lässt und einen möglichst kleinen komprimierten Querschnittsdurchmesser aufweist. Ferner ist es Aufgabe der Erfindung, ein Behandlungssystem mit einem derartigen Implantat und ein Verfahren zum Herstellen eines medizinischen Implantats anzugeben.

Erfindungsgemäß wird diese Aufgabe im Hinblick auf das medizinische Implantat durch den Gegenstand des Patentanspruchs 1, im Hinblick auf das Behandlungssystem durch den Gegenstand des Patentanspruchs 5 und im Hinblick auf das Verfahren durch den Gegenstand des Patentanspruchs 6 gelöst.

So beruht die Erfindung auf dem Gedanken, ein medizinisches Implantat mit einer radial komprimierbaren und radial expandierbaren kreiszylinderförmigen Umfangswandung anzugeben, die einstückig aus Stegen gebildet ist. Die Stege weisen (im Wesentlichen) eine kuchenstückartige Querschnittsform auf.

Die nachfolgende Erfindungsbeschreibung erläutert bevorzugte Ausgestaltungen der konstruktiven Gestaltung des medizinischen Implantats. Diese Merkmale des medizinischen Implantats gelten gleichermaßen für eine medizinische Vorrichtung, beispielsweise ein Thrombektomiedevice, ein Flow Diverter oder einen Blutfilter. Derartige Vorrichtungen weisen üblicherweise einen expandierbaren Abschnitt auf, der sich analog zu dem hier detailliert beschriebenen Implantat verhält. Der expandierbare Abschnitt ist fest, insbesondere monolithisch, mit einem Transportelement verbunden bzw. bildet einen distalen Endabschnitt des Transportelements.

Durch die kuchenstückartige Querschnittsform der Stege lässt sich das erfindungsgemäße medizinische Implantat bzw. dessen kreiszylinderförmige Umfangswandung auf einen besonders kleinen Querschnittsdurchmesser komprimieren. Das erleichtert die Zuführung des medizinischen Implantats in kleine Körperhohlorgane, beispielsweise in kleine Blutgefäße. Im implantierten Zustand bietet die Querschnittsform des medizinischen Implantats zudem einen äußerst geringen Strömungswiderstand, so dass eine Fluidströmung in den Körperhohlorganen kaum beeinflusst wird.

Überdies ist die Herstellung des erfindungsgemäßen medizinischen Implantats vereinfacht, da als Ausgangsmaterial kein Röhrchen, sondern ein Vollmaterial verwendet wird. Somit können Materialungenauigkeiten, beispielsweise eine unregelmäßige Oberflächenbeschaffenheit, die bei röhrchenförmigen Ausgangsmaterialien auf der Innenumfangsfläche auftreten können, vermieden werden. Das erfindungsgemäße medizinische Implantat weist daher eine besonders hohe Fertigungsgenauigkeit auf.

Erfindungsgemäß weisen die Stege der kreiszylinderförmigen Umfangswandung jeweils eine gekrümmte Außenfläche auf, die im komprimierten Zustand der Umfangswandung eine äußere Zylindermantelfläche der Umfangswandung bildet. In diesem Zusammenhang wird darauf hingewiesen, dass der radial komprimierte Zustand der Umfangswandung den maximal komprimierten Zustand beschreibt, soweit nichts anderes angegeben ist. Entsprechend bezeichnet der radial expandierbare Zustand der kreiszylinderförmigen Umfangswandung den maximal expandierten Zustand, falls nichts anderes angegeben ist.

Durch die gekrümmte Außenfläche der Stege, deren Krümmungsradius (im Wesentlichen) dem Krümmungsradius der äußeren Zylindermantelfläche der Umfangswandung entspricht, wenn die Umfangswandung im komprimierten Zustand vorliegt, wird eine besonders leichte Zuführung des Implantats über eine Zuführeinrichtung erreicht. Im komprimierten Zustand weist die Umfangswandung eine gleichmäßige, kreiszylinderförmige Mantelfläche auf, so dass sich das Implantat leicht durch den kreiszylinderförmigen, inneren Durchgangskanal einer Zuführeinrichtung, beispielsweise eines Katheters, führen lässt.

Die Stege der Umfangswandung weisen erfindungsgemäß jeweils zwei zur Längsachse der Umfangswandung konvergierende Seitenflächen auf. Die Seitenflächen erstrecken sich parallel zu einem Radius der Umfangswandung.

Die Seitenflächen der einzelnen Stege verlaufen vorzugsweise derart konvergierend in Richtung der Längsachse der Umfangswandung, dass der jeweilige Steg im Querschnitt im Wesentlichen eine Spitze bildet, die in Richtung des Zentrums der Umfangswandung zeigt. Die Spitze entspricht der Kuchenstückspitze der kuchenstückartigen Querschnittsform. Vorzugsweise erstrecken sich die Seitenflächen parallel zum Radius der Umfangswandung, insbesondere entlang des Radius der Umfangswandung. Das hat den Vorteil, dass die Seitenflächen benachbarter Stege im komprimierten Zustand der Umfangswandung aneinander flächig anliegen können und sich somit die Stege vollflächig gegeneinander abstützen können. Das erhöht die Stabilität der Umfangswandung im komprimierten Zustand.

Alternativ kann vorgesehen sein, dass die Seitenflächen unmittelbar benachbarter Stege in Richtung zum Außenumfang der Umfangswandung zueinander konvergieren. Unbeschadet davon, wie die Seitenflächen unmittelbar benachbarter Stege zueinander ausgerichtet sind, konvergieren die Stege eines einzelnen Stegs in Richtung zur Längsachse der Umfangswandung.

Erfindungsgemäß weisen die Stege bei dem erfindungsgemäßen Implantat bzw. der erfindungsgemäßen Vorrichtung jeweils eine (einzelne) Innenkante auf, die die Seitenflächen verbindet. Die Innenkante ist radial innerhalb der äußeren Zylindermantelfläche angeordnet und bildet eine Spitze der kuchenstückartigen Querschnittsform der Stege.

Mit anderen Worten treffen sich die zur Längsachse konvergierenden Seitenflächen an der Innenkante der Stege. Die Innenkante erstreckt sich vorzugsweise in Längsrichtung der Umfangswandung bzw. weist zumindest eine Hauptrichtungskomponente auf, die in Längsrichtung der Umfangswandung orientiert ist.

Bei einer bevorzugten Ausführungsform des medizinischen Implantats begrenzen die Stege, insbesondere die Innenkanten der Umfangswandung einen kreiszylinderförmigen Durchgangskanal. Das Verhältnis zwischen einem Innendurchmesser des Durchgangskanals und einem Außendurchmesser der Umfangswandung, insbesondere der Zylindermantelfläche, kann im komprimierten Zustand der Umfangswandung höchstens 1/20, insbesondere höchstens 1/10, insbesondere höchstens 1/7, insbesondere höchstens 1/2, betragen. Im expandierten Zustand der Umfangswanderung kann das Verhältnis zwischen Innendurchmesser und Außendurchmesser höchstens 1/100, insbesondere höchstens 1/80, insbesondere höchstens 1/60, insbesondere höchstens 1/40, betragen. Die Stege der Umfangswandung können eine Stegdicke bzw. die Umfangswandung kann eine Wandstärke aufweisen, die höchstens 0,15 mm, insbesondere höchstens 0,1 mm, insbesondere höchstens 0,075 mm, insbesondere höchstens 0,05 mm, insbesondere höchstens 0,03 mm, beträgt. Mit den zuvor genannten Werten sind medizinische Implantate herstellbar, die einerseits eine ausreichende Wandstärke aufweisen, um ein Blutgefäß sicher zu stützen und andererseits einen äußerst kleinen Querschnittsdurchmesser im komprimierten Zustand aufweisen, um in kleine Blutgefäße, beispielsweise cerebrale Blutgefäße, eingesetzt zu werden.

Vorzugsweise sind alle Stege der Umfangswandung, die in Umfangsrichtung und/oder Längsrichtung benachbart zueinander angeordnet sind, gleichartig ausgebildet. Insbesondere sind alle Stege der Umfangswandung gleichartig ausgebildet, weisen also eine kuchenstückartige Querschnittsform auf. Dabei kann die Umfangswandung grundsätzlich aus mehreren Stegen gebildet sein, die in Umfangsrichtung zueinander gleichmäßig beabstandet angeordnet sind. Insbesondere kann die Umfangswandung mehrere Längsabschnitte aufweisen, die jeweils durch eine Reihe von in Umfangsrichtung benachbart angeordneten Stegen umfassen. Die Stege in Längsrichtung benachbarter Längsabschnitte sind vorzugsweise in Umfangsrichtung zueinander versetzt angeordnet, insbesondere so dass jeweils ein Steg eines ersten Längsabschnitts mit zwei Stegen eines benachbarten zweiten Längsabschnitts der Umfangswandung verbunden ist.

Bei der Erfindung ist ferner vorgesehen, dass die Stege jeweils ein Verbundmaterial aufweisen, das aus wenigstens zwei Materialkomponenten gebildet ist. Die zwei Materialkomponenten weisen unterschiedliche Röntgensichtbarkeit und/oder unterschiedliche elektrische Leitfähigkeit auf.

Insbesondere kann eine erste Materialkomponente eine höhere Röntgensichtbarkeit oder elektrische Leitfähigkeit als eine zweite Materialkomponente aufweisen. Durch die Verwendung eines Verbundmaterials kann eine vorteilhafte Erhöhung der Röntgensichtbarkeit erreicht werden, obwohl die Stege wegen der kuchenstückartigen Querschnittsform eine reduzierte, sichtbare Fläche bieten. Durch eine geeignete Materialkombination kann dies ausgeglichen werden. Beispielweise kann die Umfangswandung durch Laserschneiden aus einem Verbunddraht hergestellt sein, der einen Kerndraht aus einer ersten Materialkomponente und eine Umhüllung aus einer zweiten Materialkomponente aufweist. Der Kerndraht kann eine höhere Röntgensichtbarkeit als die Umhüllung aufweisen. Beispielsweise kann der Kerndraht Platin oder eine Platinlegierung oder Tantal oder eine Tantallegierung aufweisen. Die Verwendung von Platin ist insbesondere zur Erhöhung der elektrischen Leitfähigkeit vorteilhaft. Dabei kann auch die Umhüllung aus Platin oder einer Platinlegierung bestehen. Die Umhüllung kann eine Nickel-Titan-Legierung aufweisen, wobei die Umhüllung im Wesentlichen tragende Eigenschaften hat, also die mechanische Stabilität der Umfangswandung gestaltet. Andere Materialkombinationen sind möglich.

Gemäß einem nebengeordneten Aspekt beruht die Erfindung auf dem Gedanken, ein Behandlungssystem mit einem zuvor beschriebenen Implantat oder der Vorrichtung und einer Zuführeinrichtung zur Zufuhr des Implantats in ein Körperhohlorgan, insbesondere ein Blutgefäß, anzugeben. Die Zuführeinrichtung kann insbesondere als Katheter ausgebildet sein, der zur Zufuhr in den menschlichen Körper angepasst ist. Das Implantat kann bei dem Behandlungssystem in der Zuführeinrichtung angeordnet sein. Alternativ ist es möglich, dass das Behandlungssystem als Sterilset vorliegt, bei dem sich das Implantat mit der Zuführeinrichtung eine Sterilgutverpackung teilt. Die Zuführeinrichtung kann auch eine Schleuse bilden, die mit einem Katheter verbindbar ist, um die Vorrichtung oder das Implantat an den Katheter zu übergeben.

Ein weiterer nebengeordneter Aspekt der Erfindung betrifft ein Verfahren zur Herstellung eines medizinischen Implantats, insbesondere des zuvor beschriebenen Implantats, aus einem kreiszylinderförmigen Vollmaterial. Das Vollmaterial wird in wenigstens einem Längsabschnitt mit einem Schneidwerkzeug radial vollständig durchtrennt, so dass eine radial komprimierbare und radial expandierbare Umfangswandung aus Stegen mit einer kuchenstückartigen Querschnittsform gebildet wird.

Das erfindungsgemäße Verfahren ermöglicht eine erhebliche Erleichterung bei der Herstellung von Implantaten, die zur Zufuhr in kleine Blutgefäße, beispielsweise cerebrale Blutgefäße, angepasst sind. Mit dem erfindungsgemäßen Verfahren wird die Verwendung von röhrchenförmigen Ausgangsmaterialien vermieden. Fertigungstoleranzen, die sich auf den Innendurchmesser von röhrchenförmigen Ausgangsmaterialen auswirken, spielen daher bei dem erfindungsgemäßen Verfahren keine Rolle. Somit ermöglicht das Verfahren eine besonders genaue Herstellung von Umfangswandungen.

Bei dem erfindungsgemäßen Verfahren werden mit dem Schneidwerkzeug Längsschlitze gebildet, die das Vollmaterial radial vollständig durchdringen. Insbesondere weisen die Längsschlitze eine Länge auf, die kleiner als eine Gesamtlänge des Vollmaterials ist. Konkret ist vorgesehen, das Vollmaterial bzw. Ausgangsmaterial (Vollzylinder) in Längsrichtung, d.h. entlang der Längsachse, in mehrere Abschnitte zu unterteilen. In jeden der Längsabschnitte kann wenigstens ein Längsschlitz eingebracht werden, wobei die Längsschlitze benachbarter Längsabschnitte in Umfangsrichtung des Vollmaterials versetzt zueinander angeordnet werden. Dadurch wird sichergestellt, dass eine zusammenhängende Umfangswandung hergestellt wird.

Vorzugsweise werden entlang einer Längsachse des Vollmaterials mehrere Längsschlitze ausgeformt, die in Umfangsrichtung versetzt zueinander angeordnet sind.

Die Längsschlitze werden vorzugsweise in Umfangsrichtung in gleichmäßigen Abständen zueinander in das Vollmaterial eingebracht. Das zwischen den Längsschlitzen stehenbleibende Material bildet die Stege der Umfangswandung. Indem die Längsschlitze in gleichmäßigen Abständen zueinander über den Umfang verteilt angeordnet werden, werden gleichmäßige Stege freigelegt. Für das so hergestellte Implantat ergibt sich der Vorteil, dass durch die Identität der Stege in Umfangsrichtung auch die Radialkraft, die das Implantat auf eine Gefäßwand eines Körperhohlorgans, beispielsweise eines Blutgefäßes, ausübt, gleichmäßig über den gesamten Umfang ist.

Vorzugsweise ist das Schneidwerkzeug ein Laserstrahl. Damit werden besonders exakte und kleine Längsschlitze in das Vollmaterial eingebracht. Insbesondere ermöglicht der Laserstrahl die Bearbeitung von Vollmaterial, das einen sehr kleinen Querschnittsdurchmesser aufweist.

Der Laserstrahl kann derart geführt bzw. fokussiert sein, dass sich im Wesentlichen parallele Schnittkanten ergeben. Damit werden Stege freigelegt, deren Seitenflächen parallel zum Radius der Umfangswandung im komprimierten Zustand ausgerichtet sind. Dies begünstigt eine vollflächige Anlage der Seitenflächen benachbarter Stege im komprimierten Zustand der Umfangswandung. Alternativ kann der Laserstrahl eine zum Zentrum des Vollmaterials sich aufweitende Strahlführung aufweisen. Dadurch wird erreicht, dass die Innenkanten der Stege im komprimierten Zustand der Umfangswandung voneinander beabstandet angeordnet sind. Es besteht somit keine vollflächige, sondern vielmehr linienartige Berührung zwischen den Stegen. Insbesondere berühren sich die Stege im Bereich des Außenumfangs der Umfangswandung, wenn die Umfangswandung komprimiert ist.

In bevorzugter Weise ist vorgesehen, dass die Stege der Umfangswandung bzw. allgemein die Umfangswandung anschließend, insbesondere nach der Bearbeitung mit dem Schneidwerkzeug, poliert werden bzw. wird. Auf diese Weise wird eine besonders glatte Oberfläche der Stege hergestellt, so dass der Einfluss des Implantats auf eine Fluidströmung in einem Blutgefäß weiter reduziert wird.

Das Vollmaterial, aus welchem die expandierbare Umfangswandung gebildet wird, ist ein Verbundmaterial, das wenigstens zwei Materialkomponenten aufweist. Die Materialkomponenten weisen unterschiedliche Röntgensichtbarkeiten und/oder elektrische Leitfähigkeiten auf. Vorzugsweise weist eine erste Materialkomponente eine höhere Röntgensichtbarkeit und/oder elektrische Leitfähigkeit als eine zweite Materialkomponente auf. Durch das Schneiden des Verbundmaterials entstehen einzelne Stege, die jeweils aus wenigstens zwei Schichten aufgebaut sind, wobei jede Schicht eine Materialkomponente bildet. Vorzugsweise sind die Schichten bzw. Materialkomponenten mit Bezug auf eine Längsachse der Umfangswandung konzentrisch zueinander angeordnet.

Die Erfindung wird im Folgenden anhand von Ausführungsbeispielen unter Bezugnahme auf die beigefügten, schematischen Zeichnungen näher erläutert. Darin zeigen
Fig. 1-3 jeweils eine Querschnittsansicht eines medizinischen Implantats nach einem bevorzugten Ausführungsbeispiel in unterschiedlichen Herstellungsstadien;
Fig. 4 eine kombinierte Querschnittsdarstellung des medizinischen Implantats gemäß Fig. 1-3 sowohl im Herstellzustand, als auch im vollständig expandierten Zustand;
Fig. 5 eine Querschnittsansicht des medizinischen Implantats gemäß Fig. 1-4 unmittelbar nach der schneidenden Bearbeitung eines Vollmaterials, das als Ausgangsmaterial für die Umfangswandung des medizinischen Implantats dient;
Fig. 6 eine Querschnittsansicht durch das medizinische Implantat gemäß Fig. 1-5 nach einem Endbearbeitungsschritt, insbesondere einem Polierschritt;
Fig. 6a eine Querschnittsansicht eines einzelnen Stegs der Umfangswandung des medizinischen Implantats gemäß Fig. 6; und
Fig. 7 eine Querschnittsdarstellung durch das medizinische Implantat gemäß Fig. 6 im vollständig komprimierten Zustand;
Fig. 8 und 9 eine Querschnittsansicht einer erfindungsgemäßen medizinischen Vorrichtung nach einem weiteren bevorzugten Ausführungsbeispiel, wobei bei der Herstellung ein Laserstrahl mit zum Zentrum des Vollmaterials sich aufweitender Strahlführung eingesetzt wird;
Fig. 10 eine perspektivische Ansicht einer erfindungsgemäßen medizinischen Vorrichtung nach einem bevorzugten Ausführungsbeispiel;
Fig. 11 eine Querschnittsansicht einer Umfangswandung der Erfindung aus einem Verbundmaterial gemäß einem bevorzugten Ausführungsbeispiel;
Fig. 12 eine Querschnittsansicht eines Stegs der Umfangswandung gemäß Fig. 11;
Fig. 13 eine Draufsicht auf einen Teil der Umfangswandung gemäß Fig. 11;
Fig. 14a-c Schnittdarstellungen der Umfangswandung gemäß Fig. 13; und
Fig. 15 eine Röntgendarstellung der Umfangswandung gemäß Fig. 13.

Die nachfolgend näher beschriebenen Ausführungsbeispiele zeigen jeweils ein medizinisches Implantat, insbesondere einen Stent, der eine kreiszylinderförmige Umfangswandung 10 aufweist. Die kreiszylinderförmige Umfangswandung 10 begrenzt einen Durchgangskanal 17, der sich längsaxial durch die gesamte Umfangswandung 10 erstreckt. Das medizinische Implantat bzw. der Stent weist also zwei axiale, offene Enden auf. Die Umfangswandung 10 des Implantats ist aus Stegen 11 gebildet, die einstückig miteinander verbunden sind. Insgesamt ist die Umfangswandung 10 einstückig, vorzugsweise aus demselben Material, ausgebildet. Bevorzugte Materialien sind Metalle, insbesondere Formgedächtnislegierungen. Besonders bevorzugt ist der Einsatz einer Nickel-Titan-Legierung, wie Nitinol, um eine selbstexpandierbare Umfangswandung 10 bereitzustellen.

Generell ist die Umfangswandung 10 radial komprimierbar und expandierbar. Das bedeutet, dass die Umfangswandung 10 von einem maximal kleinen Querschnittsdurchmesser zu einem maximal großen Querschnittsdurchmesser aufweitbar ist. Die Aufweitung der Umfangswandung 10 erfolgt vorzugsweise selbsttätig, insbesondere aufgrund von Formgedächtniseigenschaften. Das Implantat bzw. der Stent kommt insbesondere in Blutgefäßen zum Einsatz. Dabei bildet das Implantat im Wesentlichen eine Gefäßstütze, die dazu dient, das Blutgefäß offen zu halten oder zu erweitern.

Bei allen Ausführungsbeispielen des erfindungsgemäßen Implantats ist vorgesehen, dass die Stege 11 der Umfangswandung 10 eine kuchenstückartige Querschnittsform aufweisen. Mit anderen Worten bilden die Querschnittsflächen der einzelnen Stege 11 ein Kreissegment. Die Begriffe "kuchenstückartig" und "kreissegmentartig" sind insofern austauschbar. Alle kreissegmentförmigen Querschnittsflächen der Stege 11 bilden gemeinsam die kreisrunde Gesamtquerschnittsfläche der Umfangswandung 10, wenn die Umfangswandung 10 im komprimierten Zustand vorliegt. Dies gilt zumindest innerhalb von Toleranzbereichen, die dadurch entstehen, dass durch ein Schneidwerkzeug eine geringe Menge an Material abgetragen wird.

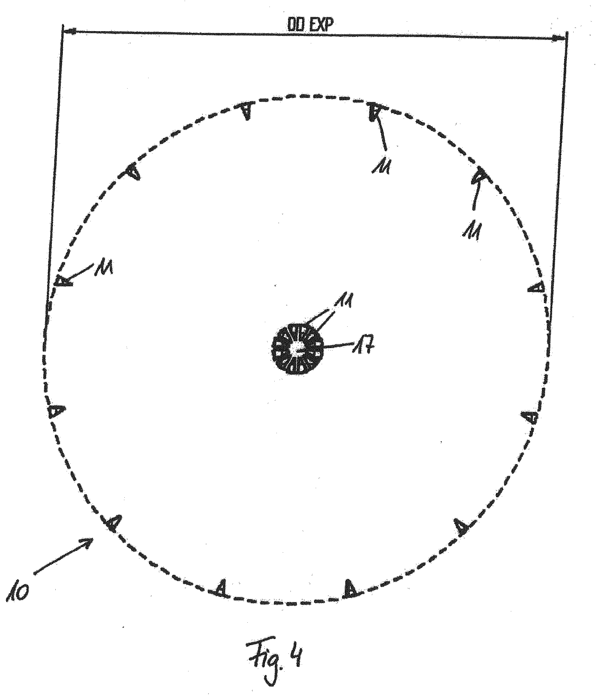

Die Stege 11 weisen im Allgemeinen eine Außenfläche 12 auf. Die Außenfläche 12 erstreckt sich entlang des Umfangs der Umfangswandung 10. Die Außenfläche 12 ist gekrümmt. Insbesondere ist die Außenfläche 12 derart gekrümmt, dass ihr Krümmungsradius dem Krümmungsradius einer Zylindermantelfläche 13 der Umfangswandung 10 entspricht. Konkret weist die Umfangswandung 10 im vollständig komprimierten Zustand einen Außendurchmesser auf, der dem Außendurchmesser der Stege 11 entspricht. Die Außenflächen 12 der Stege 11 verlaufen entlang der Zylindermantelfläche 13 der Umfangswandung 10. Die durch die Stege 11 aufgespannte Zylindermantelfläche 13 ist in Fig. 4 durch eine gestrichelte Umfangslinie dargestellt. Die gestrichelte Umfanglinie dient nur der graphischen Veranschaulichung und bildet kein reales Bauelement des Implantats.

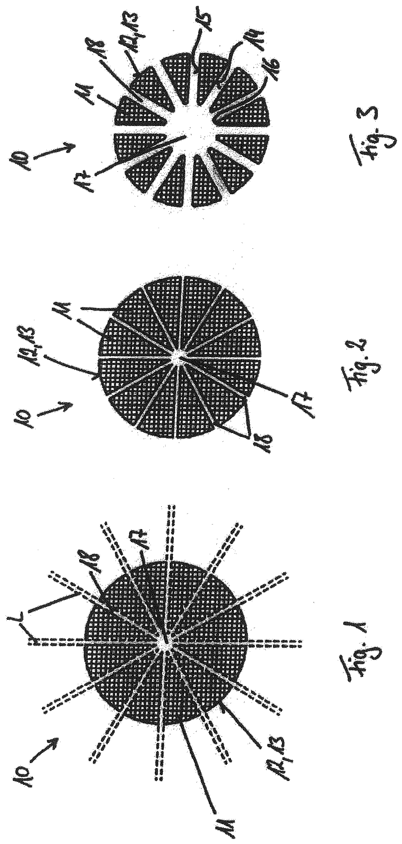

Ferner umfassen die Stege 11 zwei Seitenflächen 14, 15, die von der Außenfläche 12 in Richtung zum Zentrum, insbesondere zu einer Längsachse, der Umfangswandung 10 verlaufen. Die Seitenflächen 14, 15 konvergieren in Richtung zur Längsachse der Umfangswandung 10. Die Längsachse der Umfangswandung 10 entspricht der Zylinderachse der kreiszylinderförmigen Umfangswandung 10. Vorzugsweise, wie in den Fig. 1-3 dargestellt ist, verlaufen die Seitenflächen 14, 15 der Stege 11 im Wesentlichen parallel zu einem Radius der Umfangswandung 10. Daraus ergibt sich, dass die Seitenflächen 14, 15 benachbarter Stege 11 im vollständig komprimierten Zustand der Umfangswandung 10 im Wesentlichen vollflächig aneinander anliegen.

Konkret weist jeder Steg 11 eine erste Seitenfläche 14 und eine zweite Seitenfläche 15 auf. Im vollständig komprimierten Zustand liegt die erste Seitenfläche 14 eines Stegs 11 auf der zweiten Seitenfläche 15 eines unmittelbar in Umfangsrichtung benachbarten Stegs 11 auf. Ebenso liegt die zweite Seitenfläche 15 des Stegs 11 vollflächig an einer ersten Seitenfläche 14 eines in Gegenumfangsrichtung unmittelbar benachbarten Stegs 11 an. Die Stege 11 können im Idealfall vollflächigen Kontakt zueinander haben.

In diesem Zusammenhang wird darauf hingewiesen, dass als vollflächiger Kontakt zwischen den Seitenflächen 14, 15 auch ein Kontakt verstanden wird, der sich ergibt, wenn die Stege durch Einschneiden mit einem parallel ausgerichteten Schneidwerkzeug, beispielsweise einem Laserstrahl, freigelegt werden. Es ist klar, dass die Seitenflächen 14, 15 benachbarter Stege 11 in diesem Fall parallel zueinander verlaufen und bei weiterer radialer Kompression zum Ausgleich der in das Vollmaterial eingebrachten Schlitze sich minimal winklig zueinander anstellen. Eine minimale Abweichung von einer idealen Parallelität kann sich auch durch Nachbearbeitungsprozesse, beispielsweise inhomogene Polierprozesse, einstellen. Im Rahmen der Anmeldung wird dies ebenfalls als vollflächiger Kontakt zwischen den ersten und zweiten Seitenflächen 14, 15 verstanden. Generell berühren sich die Stege 11 im vollständig komprimierten Zustand zumindest teilweise.

Die Seitenflächen 14, 15 eines einzelnen Stegs konvergieren zueinander in Richtung zur Längsachse der Umfangswandung 10 und sind durch eine Innenkante 16 miteinander verbunden. Die Innenkante 16 kann eine flächige Erstreckung aufweisen und umfasst im Sinne der Anmeldung auch abgerundete Flächenabschnitte, die die Seitenflächen 14, 15 miteinander verbinden. Die Innenkante 16 erstreckt sich im Wesentlichen in Längsrichtung der Umfangswandung 10. Zumindest weist die Innenkante 16 eine Hauptrichtungskomponente auf, die sich in Längsrichtung der Umfangswandung 10 erstreckt. Die Innenkante 16 bildet im Wesentlichen eine Spitze der kuchenstückartigen Querschnittsform der Stege 11. Die Innenkante 16 begrenzt ferner einen Durchgangskanal, der sowohl im komprimierten Zustand, als auch im expandierten Zustand der Umfangswandung 10 erkennbar ist.

Dabei ist anzumerken, dass der Durchgangskanal 17 im komprimierten Zustand der Umfangswandung 10 (ausschließlich) aufgrund des nachfolgend näher beschriebenen Herstellungsverfahrens entsteht. Der Durchgangskanal 17 liegt also vor der Ausformung der Stege 11 nicht vor. Vielmehr ist das Ausgangsmaterial, das zur Bildung der Umfangswandung 10 eingesetzt wird, als Vollmaterial ausgebildet. Mit anderen Worten weist das Ausgangsmaterial vor der Bearbeitung zur Ausformung der Stege 11 einen vollflächig gefüllten, kreisförmigen Querschnitt auf. Das Ausgangsmaterial ist also durch einen Vollzylinder gebildet.

Die Herstellung des Implantats erfolgt durch eine schneidende Bearbeitung, vorzugsweise durch einen Laserstrahl, eines vollzylindrischen Ausgangsmaterials. Das Ausgangsmaterial ist vorzugsweise ein Metall, insbesondere eine Formgedächtnislegierung. Konkret kann das Ausgangsmaterial ein Volldraht sein. Fig. 1 zeigt einen ersten Schritt des Herstellungsverfahrens. Dabei wird der Volldraht, der einen kreisförmigen Querschnitt aufweist, entlang des Radius durch den Laserstrahl L konturiert, insbesondere vollständig durchtrennt. In das Ausgangsmaterial werden mehrere Längsschlitze 18 eingebracht, so dass die Stege 11 der Umfangswandung 10 freigeschnitten werden. Der Laserstrahl L durchdringt das Ausgangsmaterial radial, insbesondere entlang mehrerer Radien. Konkret kann das Ausgangsmaterial durch den Laserstrahl L sternförmig durchtrennt werden. Auf diese Weise bilden sich die kuchenstückartigen bzw. kreissegmentförmigen Stege 11.

Bei dem Ausführungsbeispiel gemäß Fig. 1 wird der Laserstrahl an zwölf unterschiedlichen Radien des Ausgangsmaterials bzw. der Umfangswandung 10 angesetzt, so dass sich insgesamt zwölf über den Umfang verteilte Längsschlitze 18 ergeben. Der Materialabtrag erfolgt also bis zur Mitte des Ausgangsmaterials. So wird eine größere Schnitttiefe vermieden, die zu einem ungenauen Schnittverlauf führen kann. Dies erhöht die Genauigkeit und Qualität des Herstellungsprozesses. Auf diese Weise werden zwölf Stege 11 freigelegt, die auf dem Umfang der Umfangswandung 10 verteilt angeordnet sind. Der Abstand zwischen den Radien, entlang derer der Laserstrahl L das Material schneidet, ist vorzugsweise jeweils gleich. Die Längsschlitze 18 werden also in regelmäßigen Abständen über den Umfang der Umfangswandung 10 verteilt eingebracht. Im Schnittpunkt der Längsschlitze 18, d.h. entlang der Längsachse der Umfangswandung 10 entsteht dabei aufgrund der Breite bzw. des Durchmessers des Laserstrahls L der Durchgangskanal 17. Der Durchgangskanal 17 weist im vollständig komprimierten Zustand der Umfangswandung 10 einen Innenumfang auf, der einem Vielfachen der Strahlbreite des Laserstrahls L entspricht. Bei dem Ausführungsbeispiel gemäß Fig. 1 entspricht der Umfang des Durchgangskanals 17 im Wesentlichen dem zwölffachen der Strahlbreite des Laserstrahls L. Dies beinhaltet die Breite der Innenkanten 16, die ebenfalls zum Umfang des Durchgangskanals 16 beitragen.

Fig. 2 zeigt das medizinische Implantat nach der Bearbeitung mit dem Schneidwerkzeug. Gut erkennbar sind die kuchenstückartigen Stege 11 der Umfangswandung 10 und der Durchgangskanal 17, der sich durch das Herstellungsverfahren ergibt.

Nach dem Einsatz des Schneidwerkzeugs, insbesondere nach der Laserkonturierung, werden die Stege 11 der Umfangswandung 10 vorzugsweise nachbearbeitet, um eine gewünschte Oberflächenbeschaffenheit aufzuweisen. Vorzugsweise werden die Stege 11 der Umfangswandung 10 poliert oder geätzt, um Oberflächenungenauigkeiten bzw. Rauheiten auszugleichen. Dabei verlieren die Stege 11 weiteres Material, so dass sich die Querschnittsfläche der Stege 11 insgesamt reduziert. Fig. 3 zeigt die Umfangswandung 10 mit Stegen 11 nach dem Schritt des Polierens.

Fig. 4 macht das Verhalten der Umfangswandung 10 im Gebrauch deutlich. Zum besseren Verständnis sind in Fig. 4 zwei Querschnittsansichten der Umfangswandung 10 überlagert dargestellt. Einerseits zeigt Fig. 4 eine Querschnittsansicht der Umfangswandung 10 bzw. allgemein des Implantats oder Stents im vollständig komprimierten Zustand. Insofern entspricht die innere Darstellung in Fig. 4 der Darstellung gemäß Fig. 3. Ferner zeigt Fig. 4 eine Querschnittsansicht der Umfangswandung 10 im vollständig expandierten Zustand. Die überlagerte Darstellung, bei der die vollständig komprimierte und die vollständig expandierte Umfangswandung 10 konzentrisch zueinander angeordnet sind, verdeutlicht anschaulich, dass das erfindungsgemäße Implantat von einem vergleichsweise großen expandierten Querschnittsdurchmesser auf einen äußerst kleinen, komprimierten Querschnittsdurchmesser komprimierbar ist. Somit kann das erfindungsgemäße Implantat über kleine Zuführeinrichtungen an den Behandlungsort geführt werden.

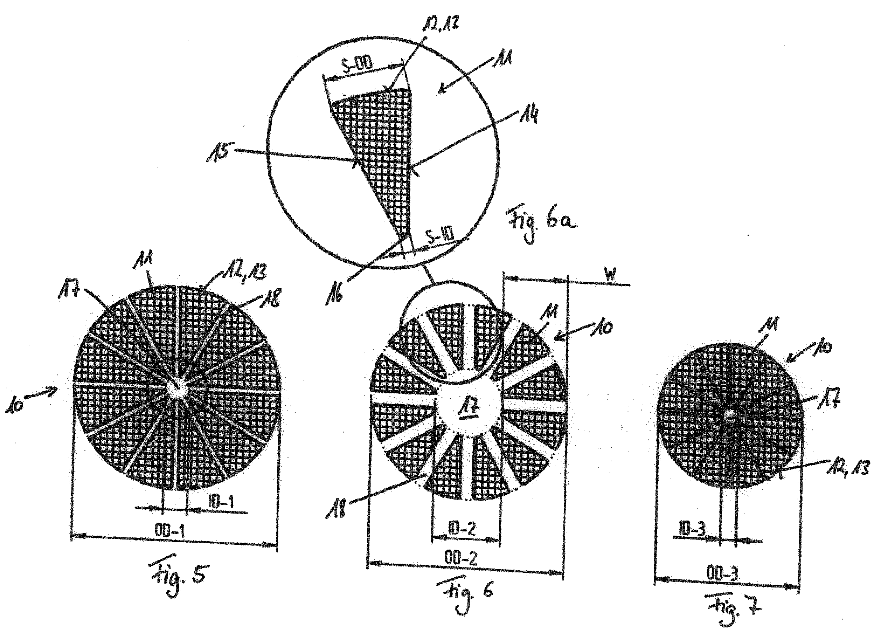

Die bevorzugten Dimensionen des medizinischen Implantats werden durch Betrachtung der Fig. 5-7 deutlich. Darin sind unterschiedliche Maße des Implantats dargestellt.

Fig. 5 zeigt die Umfangswandung 10 bzw. das Implantat im komprimierten Zustand unmittelbar nach der Bearbeitung mit dem Schneidwerkzeug, insbesondere dem Laserstrahl L. In diesem Herstellzustand weist die Umfangswandung 10 einen Außendurchmesser OD-1 auf. Der Durchgangskanal 17, der sich durch die im Zentrum treffenden Längsschlitze 18 ergibt, weist einen Innendurchmesser ID-1 auf. Der Innendurchmesser ID-1 und der Außendurchmesser OD-1 werden also vor der Nachbearbeitung, insbesondere vor der Oberflächenbearbeitung der Stege 11, konkret dem Polieren der Stege 11, ermittelt.

Der Durchgangskanal 17 weist vorzugsweise einen Innendurchmesser ID-1 auf, der höchstens 0,1 mm, insbesondere höchstens 0,06, insbesondere höchstens 0,045 mm, insbesondere höchstens 0,03 mm, beträgt. Der Innendurchmesser ID-1 des Durchgangskanals 17 unmittelbar nach dem Einbringen der Längsschlitze 18 in das Ausgangsmaterial wird insbesondere durch die Strahlweite bzw. die Breite des Laserstrahls L und/oder die Strahlform des Laserstrahls L beeinflusst.

Die Umfangswandung 10 weist in diesem Stadium des Herstellprozesses, d.h. unmittelbar nach dem Einbringen der Laserschlitze 18 in das Ausgangsmaterial, einen Außendurchmesser OD-1 auf, der höchstens 1,1 mm, insbesondere höchstens 0,9 mm, insbesondere höchstens 0,8 mm, insbesondere höchstens 0,5 mm, insbesondere höchstens 0,4 mm, insbesondere höchstens 0,3 mm, insbesondere höchstens 0,2 mm, beträgt. Der Außendurchmesser OD-1 der Umfangswandung 10 wird insbesondere durch die Wahl des Ausgangsmaterials beeinflusst. Konkret kann der Außendurchmesser OD-1 dem Drahtdurchmesser des verwendeten Volldrahts entsprechen, der das Ausgangsmaterial für die Herstellung der Umfangswandung 10 bildet.

Nach dem Nachbearbeitungsschritt, der die Oberflächenstruktur der Stege 11 beeinflussen soll, insbesondere nach dem Polieren der Stege 11, weist das Implantat im vollständig komprimierten Zustand veränderte Maße auf. Insbesondere kann der Durchgangskanal 17 nach dem Polieren der Stege 17 einen Innendurchmesser ID-2 aufweisen, der höchstens 0,105 mm, insbesondere höchstens 0,065 mm, insbesondere höchstens 0,05 mm, insbesondere höchstens 0,035 mm, beträgt. Wegen des Materialabtrags beim Polieren der Stege 11 vergrößert sich also der Innendurchmesser ID-2 des Durchgangskanals 17 nach dem Polieren. Umgekehrt kann sich der Außendurchmesser OD-2 der Umfangswandung 10 nach dem Polieren reduzieren. So ist vorgesehen, dass der Außendurchmesser OD-2 der Umfangswandung 10 nach dem Polieren der Stege 11 höchstens 0,9 mm, insbesondere höchstens 0,88 mm, insbesondere höchstens 0,78 mm, insbesondere höchstens 0,38 mm, insbesondere höchstens 0,28 mm, insbesondere höchstens 0,18 mm, insbesondere höchstens 0,13 mm, beträgt. Die Vergrößerung des Innendurchmessers ID-2 und des Außendurchmessers OD-2 wird durch das eingesetzte Nachbearbeitungsverfahren, insbesondere das eingesetzte Polierverfahren, beeinflusst. Bei bevorzugten Ausführungsbeispielen reduziert sich der Außendurchmesser OD-2 durch das Polieren um höchstens 0,2 mm, insbesondere höchstens 0,15 mm, insbesondere höchstens 0,1 mm, insbesondere höchstens 0,05 mm, insbesondere höchstens 0,1 mm, insbesondere höchstens 0,05 mm, insbesondere höchstens 0,02 mm. Entsprechend vergrößert sich der Innendurchmesser ID-2 des Durchgangskanals 17 nach dem Polieren vorzugsweise um höchstens 0,005 mm, insbesondere höchstens 0,0025 mm, insbesondere höchstens 0,002 mm gegenüber dem Innendurchmesser ID-1 vor dem Polieren.

Die Wandstärke W der Umfangswandung 10, die im Wesentlichen der Stegdicke der Stege 11 entspricht, beträgt bei bevorzugten Ausführungsformen höchstens 0,15 mm, insbesondere höchstens 0,1 mm, insbesondere höchstens 0,075 mm, insbesondere höchstens 0,05 mm, insbesondere höchstens 0,03 mm. Die Wandstärke W wird durch die Wahl des Ausgangsmaterials, durch die Breite bzw. Querausdehnung des Laserstrahls L und/oder durch die Strahlform des Laserstrahls L beeinflusst. Erheblichen Einfluss auf die Wandstärke W hat auch die Wahl des Nachbearbeitungsprozesses.

Fig. 6a zeigt eine Querschnittsansicht eines einzelnen Stegs 11 im Detail. Es ist erkennbar, dass der Steg 11 eine Stegbreite S-ID am Innendurchmesser des Durchgangskanals 17 und eine davon abweichende Stegbreite S-OD am Außendurchmesser der Umfangswandung 10 aufweist. Die Stegbreite S-ID am Innendurchmesser beträgt vorzugsweise höchstens 0,075 mm, insbesondere höchstens 0,05 mm, insbesondere höchstens 0,03 mm, insbesondere höchstens 0,015 mm, insbesondere höchstens 0,01 mm, insbesondere höchstens 0,009 mm, insbesondere höchstens 0,0075 mm. Einfluss auf die Stegbreite S-ID am Innendurchmesser des Durchgangskanals 17 haben insbesondere die Wahl des Ausgangsmaterials, die Strahlbreite und/oder Strahlform des Laserstrahls L, die Art und Weise der Nachbearbeitung sowie der Winkel der Flanken, d.h. der Winkel, den die beiden Seitenflächen 14, 15 des Stegs 11 miteinander einschließen. Letzterer wird insbesondere durch die Anzahl der Stege 11 über den Umfang der Umfangswandung 10 beeinflusst. Konkret weisen die erste und zweite Seitenfläche 14, 15 zueinander einen Winkel auf, der umso größer wird, je weniger Stege 11 über den Umfang der Umfangswandung 10 verteilt angeordnet sind.

Die Stegbreite S-OD am Außendurchmesser der Umfangswandung 10 beträgt bei bevorzugten Ausführungsformen höchstens 0,15 mm, insbesondere höchstens 0,1 mm, insbesondere höchstens 0,09 mm, insbesondere höchstens 0,08 mm, insbesondere höchstens 0,07 mm, insbesondere höchstens 0,05 mm, insbesondere höchstens 0,03 mm, insbesondere höchstens 0,025 mm. Die Stegbreite S-OD am Außendurchmesser der Umfangswandung 10 bzw. der Zylindermantelfläche 13 wird hauptsächlich durch die Wahl der Endbearbeitungsschritte, beispielsweise die Wahl des Polierverfahrens, und/oder den Winkel der Flanken bzw. der Seitenflächen 14, 15 zueinander, also die Anzahl der Stege 11 entlang des Umfangs der Umfangswandung 10, beeinflusst.

Vorzugsweise stehen der Innendurchmesser ID-1 und der Außendurchmesser OD-2 vor dem Nachbearbeiten bzw. Polieren der Umfangswandung 10 in einem besonderen Verhältnis zueinander, um einerseits die ausreichende Radialkraft für das Implantat bereitzustellen und andererseits einen geringstmöglichen komprimierten Querschnittsdurchmesser zu erreichen. Insbesondere kann vorgesehen sein, dass das Verhältnis ID-1/OD-1 zwischen dem Innendurchmesser ID-1 des Durchgangskanals 17 und dem Außendurchmesser OD-1 der Umfangswandung 10 bzw. der Zylindermantelfläche 13 vor dem Nachbearbeiten bzw. Polieren der Umfangswandung 10 höchstens 1/40, insbesondere höchstens 1/20, insbesondere höchstens 1/10, insbesondere höchstens 1/5, insbesondere höchstens 1/2, beträgt. Entsprechend kann vorgesehen sein, dass das Verhältnis ID-2/OD-2 zwischen dem Innendurchmesser ID-2 des Durchgangskanals 17 und dem Außendurchmesser OD-2 der Umfangswandung 10 nach dem Nachbearbeitungsschritt (nach dem Polieren), d.h. im fertiggestellten Zustand, höchstens 1/20, insbesondere höchstens 1/10, insbesondere höchstens 1/5, insbesondere 1/2, beträgt.

Hinsichtlich des Verhältnisses des Außendurchmessers OD-2 der Umfangswandung 10 im fertiggestellten Zustand, insbesondere nach dem Polieren, zur Wandstärke W, also zur Stegdicke der Stege 11, ist ein Verhältnis von W/OD-2 von höchstens 0,45, insbesondere höchstens 0,3, insbesondere höchstens 0,2, insbesondere höchstens 0,15, insbesondere höchstens 0,1, insbesondere höchstens 0,05, vorgesehen.

Vorzugsweise stehen die Stegbreite S-OD am Außendurchmesser der Umfangswandung 10 und die Stegbreite S-ID am Innendurchmesser des Durchgangskanals 17 in einem Verhältnis S-OD/S-ID zueinander, dass mindestens 2, insbesondere mindestens 5, insbesondere mindestens 10, insbesondere mindestens 20, insbesondere mindestens 50, beträgt.

Figur 7 zeigt eine Querschnittsansicht durch das Implantat bzw. den Stent gemäß Figur 6 im vollständig komprimierten Zustand. Es ist klar erkennbar, dass die Stege 11 bündig bzw. vollflächig aneinander anliegen. Insofern weist das Implantat im vollständig komprimierten Zustand nach Figur 7 einen kleineren Außendurchmesser OD-3 als im Herstellzustand gemäß Figur 6 auf. Konkret beträgt der Außendurchmesser OD-3 der Umfangswandung 10 im vollständig komprimierten Zustand vorzugsweise höchstens 0,87 mm, insbesondere höchstens 0,85 mm, insbesondere höchstens 0,75 mm, insbesondere höchstens 0,35 mm, insbesondere höchstens 0,28 mm, insbesondere höchstens 0,25 mm, insbesondere höchstens 0,15 mm, insbesondere höchstens 0,1 mm.

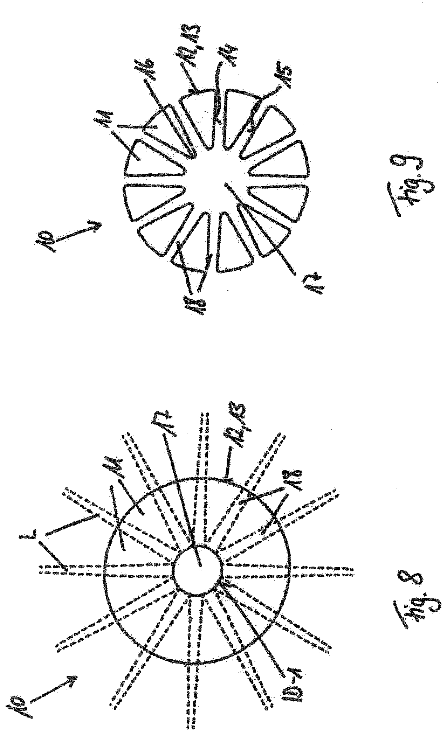

Auch der Innendurchmesser ID-3 der Umfangswandung 10 ist im vollständig komprimierten Zustand gemäß Figur 7 kleiner als im Herstellzustand gemäß Figur 6.
Im Allgemeinen kann für die Bildung der Längsschlitze 18, die das Ausgangsmaterial durchdringen, ein Laserstrahl L eingesetzt werden, der eine parallele Mit anderen Worten weist der Laserstrahl L im Wesentlichen einen konstanten Strahlquerschnitt auf. Alternativ kann vorgesehen sein, dass der Laserstrahl L einen Strahlquerschnitt aufweist, der sich in Richtung des Zentrums der Umfangswandung 10 aufweitet. Ein Ausführungsbeispiel für die Herstellung des medizinischen Implantats mit einem derartig sich zum Zentrum der Umfangswandung 10 aufweitenden Laserstrahl L ist in den Fig. 8 und 9 gezeigt.

Fig. 8 zeigt den Laserstrahl L an verschiedenen Radien der Umfangswandung 10 bzw. des Ausgangsmaterials, aus welchem die Umfangswandung 10 gebildet wird. Es ist gut erkennbar, dass durch den sich mit zunehmender Nähe zum Mittelpunkt der Umfangswandung 10 aufweitenden Laserstrahl L der Innendurchmesser D-1 des Durchgangskanals 17 im komprimierten Zustand der Umfangswandung 10 gegenüber einem Herstellungsverfahren, bei dem ein Laserstrahl L mit paralleler Strahlführung eingesetzt wird, vergrößert wird. Gleichzeitig reduziert sich das Verhältnis zwischen der Stegbreite S-OD am Außendurchmesser zur Stegbreite S-ID am Innendurchmesser gegenüber einem Herstellungsverfahren, bei dem ein Laserstrahl L mit paralleler Strahlführung eingesetzt wird. Folglich ist die Wandstärke W beim Einsatz eines Laserstrahls L mit sich aufweitender Strahlführung kleiner als bei Verwendung eines Laserstrahls L mit paralleler Strahlführung. Fig. 9 zeigt das Implantat gemäß Fig. 8, wobei die Umfangswandung 10 poliert wurde. Durch das Polieren ergibt sich die Abrundung der Innenkante 16 der Stege 11.

Im Allgemeinen gilt für alle Ausführungsbeispiele, dass das Ausgangsmaterial, insbesondere ein Volldraht, vorzugsweise in mehrere Längsabschnitte unterteilt wird. Jeder der Längsabschnitte umfasst mehrere Längsschlitze 18. Die Längsschlitze 18 werden in den Längsabschnitten vorzugsweise in regelmäßigen Abständen über den Umfang der Umfangswandung 10 verteilt angeordnet und durchdringen das Ausgangsmaterial bzw. die Umfangswandung 10 vollständig. Die Längsschlitze 18 benachbarter Umfangssegmente sind vorzugsweise versetzt zueinander angeordnet, so dass sich insgesamt eine einstückig miteinander verbundene Umfangswandung 10 ergibt. Der Versatz der Längsschlitze 18 benachbarter Längsabschnitte erfolgt vorzugsweise in Umfangsrichtung. Insbesondere entspricht die Summe der Längen der Längsschlitze 18 der Gesamtlänge der Umfangswandung 10.

Im Allgemeinen gilt für das Herstellverfahren des Implantats, dass beim Schneiden der Struktur der Umfangswandung 10 aus dem kreiszylinderförmigen Vollmaterial, insbesondere dem Volldraht, Stege mit einer kreissegmentartigen Grundfläche freigelegt bzw. ausgebildet werden. Die Stege weisen insofern eine kuchenstückartige bzw. kreissegmentartige Querschnittsform auf. Wenn das Schneiden mittels des Laserstrahls L erfolgt, verläuft der Laserstrahls L bzw. ein erster Laserstrahl L wie eine Speiche bis zum Mittelpunkt des Vollmaterial, insbesondere des Volldrahts, um eine Seitenfläche 14, 15 des Stegs 11 zu bilden. Die andere Seitenfläche 15, 14 wird durch einen bis zum Mittelpunkt des Vollmaterials, insbesondere des Volldrahts, verlaufenden zweiten Laserstrahl L gebildet, der den Strahlverlauf des ersten Laserstrahls L schneidet. Der Schnittpunkt der Laserstrahlen L liegt vorzugsweise in der Längsachse der Umfangswandung 10. Der erste und zweite Laserstrahl L können das Material gleichzeitig oder zeitlich aufeinanderfolgend bearbeiten.

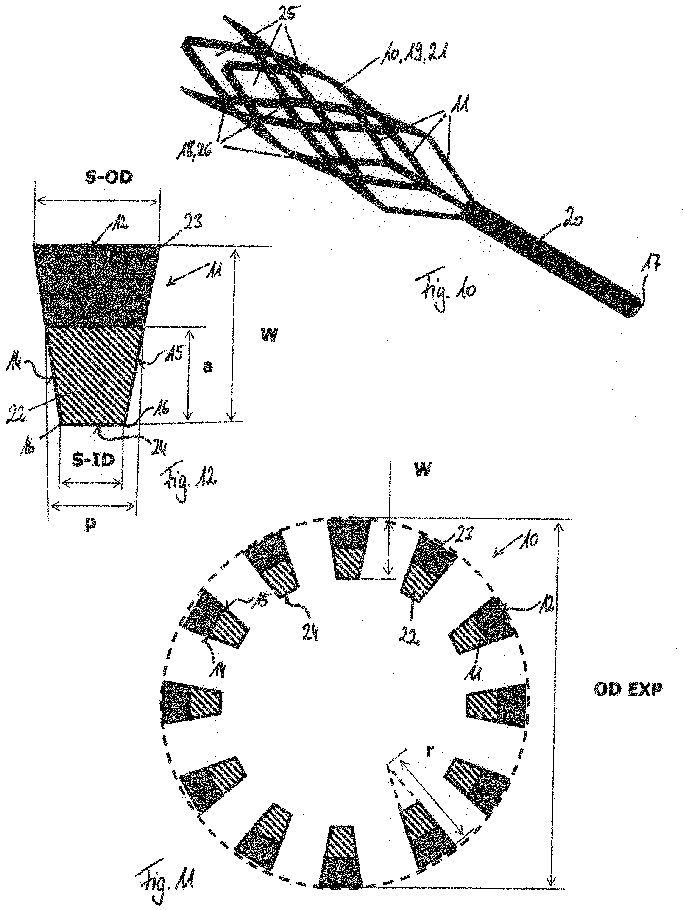

Fig. 10 zeigt eine perspektivische Darstellung einer medizinischen Vorrichtung, die einen distalen Endabschnitt 19 aufweist, der im Wesentlichen analog zu dem zuvor detailliert beschriebenen Implantat ausgebildet ist. Der distale Endabschnitt 19 ist einstückig bzw. monolithisch mit einem proximalen Transportelement 20 ausgebildet. Das Transportelement 20 ist bei dem vorliegenden Ausführungsbeispiel als Hohldraht gebildet, kann jedoch auch ein Vollmaterial aufweisen.

Der distale Endabschnitt 19 bildet eine Umfangswandung 10, die radial expandierbar ist. Die Umfangswandung 10 ist aus mehreren Stegen 11 gebildet, die kuchenstückartig geformt sind. Vorzugsweise ist die gesamte Vorrichtung gemäß Fig. 10 aus einem einzelnen Rohmaterial, beispielsweise einem Hohldraht oder einem Volldraht, gebildet. Im distalen Endabschnitt 19 werden bei der Herstellung Längsschlitze 18 eingebracht, die jeweils derart versetzt zueinander angeordnet sind, dass sich eine Gitterstruktur 21 bildet. Die Gitterstruktur 21 ist expandierbar und einstückig mit dem Transportelement 20 verbunden. Die in Fig. 10 dargestellte Vorrichtung kann beispielsweise als Thrombectomiedevice, also zum Entfernen von Thromben aus Blutgefäßen, eingesetzt werden. Weitere Einsatzmöglichkeiten für erfindungsgemäß ausgebildete Vorrichtungen sind Blutfilter. Im Allgemeinen ist die erfindungsgemäße medizinische Vorrichtung oder das erfindungsgemäße medizinische Implantat zum Einsatz in Blutgefäßen geeignet.

Fig. 11 zeigt einen Querschnitt durch die Umfangswandung 10 einer erfindungsgemäßen medizinischen Vorrichtung oder eines medizinischen Implantats. Im Wesentlichen entspricht die Darstellung gemäß Fig. 11 der Darstellung gemäß Fig. 4, wobei die Umfangswandung 10 im expandierten Zustand dargestellt ist. Im Unterschied zu den vorhergehenden Ausführungsbeispielen ist bei dem Ausführungsbeispiel gemäß Fig. 11 vorgesehen, dass die Umfangswandung aus einem Verbundmaterial gebildet ist. Das Verbundmaterial umfasst zwei oder mehr Materialkomponenten 22, 23, die sich hinsichtlich Röntgensichtbarkeit und/oder elektrischer Leitfähigkeit unterscheiden. Konkret ist vorgesehen, dass die expandierbare Umfangswandung aus einem einzigen Draht hergestellt ist, der als Verbunddraht ausgebildet ist, also ein Verbundmaterial aufweist. Insbesondere kann der Draht einen Kerndraht aus einer ersten Materialkomponente 22 und eine Umhüllung aus einer zweiten Materialkomponente 23 aufweisen. Die erste Materialkomponente 22 weist vorzugsweise eine höhere Röntgensichtbarkeit als die zweite Materialkomponente 23 auf. Auf diese Weise ergibt sich eine Umfangswandung 10, die mehrschichtig aufgebaut ist, wobei die einzelnen Schichten konzentrisch bzw. koaxial zueinander angeordnet sind.

Fig. 12 zeigt im Detail einen Querschnitt durch einen Steg 11 der Umfangswandung 10 gemäß Fig. 11. Es ist gut erkennbar, dass der Steg 11 eine erste Materialkomponente 22 und eine zweite Materialkomponente 23 aufweist. Die erste Materialkomponente 22 ist radial innerhalb der zweiten Materialkomponente 23 angeordnet. Ferner ist aus Fig. 12 gut erkennbar, dass die Seitenflächen 14, 15 des Stegs 11 zueinander konvergieren und auf einem Innenumfang der Umfangswandung 10 durch eine Innenfläche 24 miteinander verbunden sind. Mit anderen Worten bildet der Steg 11 einen kuchenstückartigen Querschnitt mit abgeflachter Spitze, wobei die abgeflachte Spitze durch die Innenfläche 24 gebildet ist. Aus Gründen der Übersichtlichkeit ist in der schematischen Darstellung des Stegs 11 gemäß Fig. 12 die Abrundung der Kanten nicht dargestellt.

Mit Bezug auf die Figuren 11 und 12 werden nachfolgend einige bevorzugte Dimensionen der Umfangswandung 10 angegeben, die generell für alle Ausführungsbeispiele der Erfindung gelten.

So weist die Umfangswandung 10 im expandierten Zustand vorzugsweise einen Außendurchmesser OD EXP auf, der zwischen 2,5 mm und 8 mm, insbesondere zwischen 3 mm und 7 mm, insbesondere zwischen 3,5 mm und 6 mm, insbesondere 4,5 mm beträgt. Das Verhältnis zwischen der Wandstärke W der Umfangswandung 10 bzw. der Stegdicke und dem Außendurchmesser OD EXP der Umfangswandung 10 im expandierten Zustand, d.h. das Verhältnis W/D, beträgt vorzugsweise zwischen 0,00625 und 0,04, insbesondere zwischen 0,008 und 0,03, insbesondere 0,01 bis 0,02.
Der Abstand zwischen der Außenfläche 12 des Stegs 11 und einem Schnittpunkt der Seitenflächen 14, 15 wird als Schnitttiefe r bezeichnet. Die Schnitttiefe r entspricht dem Verhältnis zwischen dem halben Durchmesser eines Katheters, durch welchen die Vorrichtung bzw. das Implantat geschoben werden kann, und dem Außendurchmesser der Umfangswandung 10 im expandierten Zustand. Das Verhältnis zwischen der Schnitttiefe r und dem Außendurchmesser OD EXP der Umfangswandung 10 im expandierten Zustand, d.h. das Verhältnis r/D, beträgt vorzugsweise zwischen 0,025 und 0,08, insbesondere zwischen 0,03 und 0,06, insbesondere zwischen 0,04 und 0,05. Der Außendurchmesser des Drahts, aus dem durch Laserschneiden die Umfangswandung 10 hergestellt wird, beträgt im ungeschnittenen Zustand der doppelten Schnitttiefe r. Vorzugsweise beträgt daher der Außendurchmesser des Drahts, der zur Herstellung der Umfangswandung 10 genutzt wird (2 · r) zwischen 0,2 mm und 0,5 mm, insbesondere zwischen 0,3 mm und 0,4 mm.

Anhand der Schnittansicht gemäß Fig. 12 werden im Folgenden einige Dimensionen angegeben. Dabei wird darauf hingewiesen, dass sich die Dimensionsangaben auf den Steg 11 nach dem Schritt des Elektropolierens beziehen. Mit anderen Worten weist der Steg 11 im Bereich seiner Kanten jeweils eine Abrundung auf. Die Abrundung beträgt vorzugsweise wenigstens 5 µm, insbesondere wenigstens 10 µm, insbesondere wenigstens 15 µm, jedenfalls jedoch maximal 30 µm. Fig. 12 zeigt den Steg 11 mit einer Innenfläche 24. Es ist allerdings auch möglich, dass der Steg 11 eine Innenkante 16 aufweist, die die beiden Seitenflächen 14, 15 direkt miteinander verbindet. Mit anderen Worten kann die Innenkante 16 vollständig abgerundet sein, so dass die Innenkante 16 mit einer Innenfläche 24 verschliefen ist.

Der Steg gemäß Fig. 12 weist zwei Materialkomponenten 22, 23 auf. Diese bilden jeweils eine Materialschicht, wobei die Schichtdicke a der ersten Materialkomponente 22 vorzugsweise zwischen 5 µm und 20 µm, insbesondere zwischen 6 µm und 15 µm, insbesondere zwischen 8 µm und 12 µm, vorzugsweise 10 µm, beträgt. Die gesamte Stegdicke bzw. Wandstärke W beträgt vorzugsweise zwischen 30 µm und 100 µm, insbesondere zwischen 40 µm und 80 µm, insbesondere zwischen 50 µm und 70 µm, vorzugsweise 60 µm.

Das Verhältnis zwischen der Schichtdicke a der ersten Materialkomponente 22 und der Wandstärke W, also das Verhältnis a/W, beträgt vorzugsweise zwischen 5 % und 40 %, insbesondere zwischen 10 % und 30 %, insbesondere zwischen 15 % und 25 %, vorzugsweise 20 %. Die vorgenannten Werte gelten insbesondere, wenn die erste Materialkomponente 22 ein röntgensichtbares Material aufweist bzw. eine höhere Röntgensichtbarkeit als die zweite Materialkomponente 23 umfasst. In dem dargestellten Ausführungsbeispiel ist die erste Materialkomponente 22 auf einem Innenumfang der Umfangswandung 10 angeordnet. Es ist auch möglich, dass die erste Materialkomponente, vorzugsweise einschließlich der zuvor genannten Dimensionen der Schichtdicke a, auf einem Außenumfang der Umfangswandung 10 befindet. Die Anordnung der ersten Materialkomponente 22 auf dem Außenumfang ist insbesondere bevorzugt, wenn die erste Materialkomponente 22 eine höhere elektrische Leitfähigkeit als die zweite Materialkomponente 23 aufweist. Ferner kann die erste Materialkomponente 22 eine Zwischenschicht bilden, die zwischen zwei weiteren Schichten eingebettet ist. Wenn die erste Materialkomponente 22 eine verbesserte elektrische Leitfähigkeit aufweist, kann die Zwischenschicht insbesondere ein elektrisch isolierendes Material umfassen. Die erste Materialkomponente 22 ist dann vorzugsweise auf dem Außenumfang angeordnet und durch die Zwischenschicht von der zweiten Materialkomponente 23 getrennt, die auf dem Innenumfang angeordnet ist und eine tragende Funktion übernimmt.

Vorzugsweise weist der Draht, aus dem die Umfangswandung 10 geschnitten wird, ein Volumenverhältnis zwischen der ersten Materialkomponente 22 und der zweiten Materialkomponente 23 auf, das zwischen 10 % und 50 %, insbesondere zwischen 12 % und 40 %, insbesondere zwischen 15 % und 30 %, vorzugsweise 20 % beträgt. In diesem Fall ist die erste Materialkomponente 22 mit einer höheren Röntgensichtbarkeit als die zweite Materialkomponente 23 ausgestattet.

Die Stegbreite S-OD am Außendurchmesser der Umfangswandung 10 beträgt vorzugsweise zwischen 20 µm und 75 µm, insbesondere zwischen 22 µm und 50 µm, insbesondere zwischen 25 µm und 45 µm, insbesondere zwischen 30 µm und 40 µm. Die Stegbreite S-ID am Innendurchmesser der Umfangswandung 10 beträgt vorzugsweise zwischen 10 µm und 50 µm, insbesondere zwischen 15 µm und 30 µm, insbesondere zwischen 18 µm und 25 µm. Die vorgenannten Werte entsprechen vorzugsweise der maximal möglichen Rundung am Innenumfang der Umfangswandung 10. Das Verhältnis zwischen der Stegbreite S-ID Innendurchmesser der Umfangswandung 10 zur Stegbreite S-OD an der Außenfläche 12 der Umfangswandung 10, also das Verhältnis S-ID/S-OD, ist vorzugsweise kleiner als 0,8, insbesondere kleiner als 0,6, insbesondere kleiner als 0,4, insbesondere kleiner als 0,3, aber größer als 0,1.

Fig. 12 zeigt außerdem eine Zwischenbreite p, die der Stegbreite im Bereich des Übergangs zwischen den zwei Materialkomponenten 22, 23 entspricht. Die Zwischenbreite p beträgt vorzugsweise zwischen 20 µm und 60 µm, insbesondere zwischen 25µm und 35 µm.

Das Verhältnis p/S-OD zwischen der Zwischenbreite p und der Stegbreite S-OD am Außendurchmesser der Umfangswandung 10 ist vorzugsweise kleiner als 0,8, insbesondere kleiner als 0,7, insbesondere kleiner als 0,6, insbesondere kleiner als 0,5, jedoch größer als 0,2.

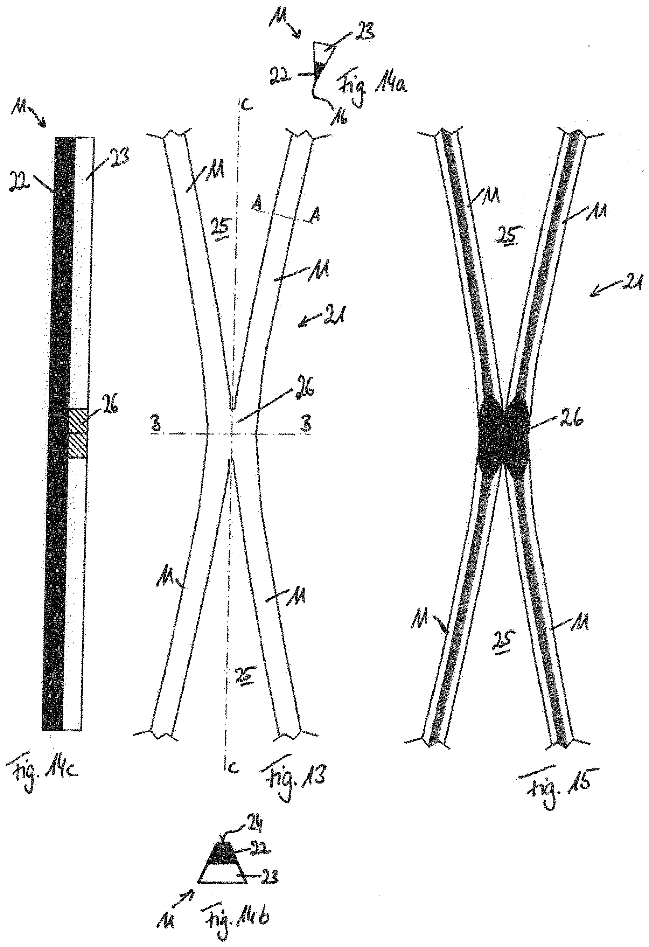

In Fig. 13 ist eine Draufsicht auf ein Teil einer Gitterstruktur 21 gezeigt, die nach dem erfindungsgemäßen Verfahren hergestellt ist. Die Gitterstruktur 21 weist mehrere Stege 11 auf, die Zellen 25 begrenzen. Die Stege 11 weisen eine kuchenstückartige Querschnittsform auf.

So zeigt Fig. 14a beispielsweise einen Querschnitt des Stegs 11 entlang der Linie A-A in Fig. 13. Es ist erkennbar, dass der Steg 11 zwei Materialkomponenten 22, 23 aufweist, wobei eine erste Materialkomponente 22 mit einer relativ höheren Röntgensichtbarkeit radial innerhalb der zweiten Materialkomponente 23 mit der relativ niedrigeren Röntgensichtbarkeit angeordnet ist. Im Wesentlichen ist die Gitterstruktur 21 bzw. Umfangswandung 10 also zweischichtig ausgebildet, wobei eine radial innere Schicht eine höhere Röntgensichtbarkeit als eine radial äußere Schicht aufweist.

Fig. 14b zeigt eine Schnittansicht durch die Gitterstruktur 21 entlang der Linie B-B in Fig. 13. Bedingt durch das Zusammentreffen mehrerer Stege 11 im Bereich einer Verbindungsstelle 26 der Gitterstruktur 21 zeigt die Querschnittsansicht gemäß Fig. 14b eine abgeflachte Spitze des an sich kuchenstückartigen Querschnitts. Fig. 14c zeigt eine Längsschnittansicht der Umfangswandung 10 entlang der Linie C-C in Fig. 13. Es ist gut erkennbar, dass die einzelnen Stege 11 vollständig aus zwei Materialkomponenten 22, 23 gebildet sind. Die beiden Materialkomponenten 22, 23 bilden gemeinsam ein Verbundmaterial.

Fig. 15 zeigt eine Röntgenaufnahme des Gitterstrukturausschnitts gemäß Fig. 13. Es ist gut erkennbar, dass die Röntgensichtbarkeit insbesondere durch das Materialvolumen der ersten Materialkomponente 22 beeinflusst wird. So ist im Bereich der Stege 11 wegen der kuchenstückartigen Querschnittsform ein geringeres Materialvolumen der ersten Materialkomponente 22 vorhanden als im Bereich der Verbindungsstelle 26. Die Verbindungsstelle 26 ist daher unter Röntgenstrahlung besser sichtbar als die Stege 11. Die Röntgensichtbarkeit kann also über die gesamte Umfangswandung 10 unterschiedlich ausgeprägt sein.

Die nachfolgende Beschreibung allgemeiner Merkmale der Erfindung gilt für alle Ausführungsbeispiele:

Im Allgemeinen wird im Rahmen der vorliegenden Anmeldung ein Implantat oder eine Vorrichtung beschrieben, die jeweils wenigstens einen Steg 11 aufweisen, der eine kuchenstückförmige Querschnittsform aufweist. Dabei ist eine Spitze der kuchenstückartigen Querschnittsform nach innen gerichtet. Mit anderen Worten konvergieren die Seitenflächen 14, 15 des Stegs 11 radial nach innen zu einer Längsachse der Umfangswandung 10. Vorzugsweise kann der Steg 11 wenigstens zwei Materialkomponenten 22, 23 aufweisen, wobei eine erste Materialkomponente 22 eine höhere Röntgensichtbarkeit als eine zweite Materialkomponente 23 aufweist. Es ist auch möglich, dass die erste Materialkomponente eine höhere elektrische Leitfähigkeit als die zweite Materialkomponente 23 aufweist. Vorzugsweise ist die erste Materialkomponente 22 mit der höheren Röntgensichtbarkeit radial innerhalb der zweiten Materialkomponente 23 angeordnet. Im Allgemeinen kann das Implantat oder die Vorrichtung mehrere Stege 11 aufweisen. Die Stege 11 bilden gemeinsam eine Umfangswandung 10 bzw. eine Gitterstruktur 21. Die Umfangswandung 10 bzw. Gitterstruktur 21 ist radial expandierbar. Vorzugsweise erstrecken sich die Stege 11 hauptsächlich in Längsrichtung der Umfangswandung 10 oder helixförmig um eine Längsachse der Umfangswandung 10. Eine derartige Gestaltung der Umfangswandung 10 ist insbesondere für Blutfilter und Thrombectomiedevices geeignet. Die Stege 11 können im Allgemeinen eine Gitterstruktur bilden, was insbesondere für den Einsatz der medizinischen Vorrichtung als Stent, Flowdiverter oder Thrombectomiedevice vorteilhaft ist.

Ferner kann ein Teil der medizinischen Vorrichtung unstrukturiert sein, also nicht durch Laserschneiden bearbeitet sein. Ein derartiger Bereich der Vorrichtung bildet vorzugsweise ein Transportelement, das im Wesentlichen durchmesserstabil ausgebildet ist. Auf diese Weise kann ein distaler Endabschnitt 19, der als expandierbare Umfangswandung 10 ausgebildet ist, an den Behandlungsort geführt werden. Eine medizinische Vorrichtung, die zum temporären Einsatz in Blutgefäßen vorgesehen ist und einen radial expandierbaren distalen Endabschnitt 19 und einen proximalen, durchmesserstabilen Abschnitt aufweist, der als Transportelement 20 dient, kann insbesondere als Thrombectomiedevice, Blutfilter oder auch als Elektrode zur Stimulation oder Ablation eingesetzt werden.

Es ist ferner möglich, mehrere, expandierbare Umfangswandungen 10 miteinander zu koppeln. So kann eine medizinische Vorrichtung gebildet werden, die aus mehreren, längsaxial hintereinander angeordneten, expandierbaren Umfangswandungen 10 gebildet ist. Zwischen den einzelnen Umfangswandungen 10 können gelenkartige Elemente angeordnet sein. Die gelenkartigen Elemente können durch durchmesserstabile Abschnitte gebildet sein. Vorzugsweise ist die gesamte medizinische Vorrichtung monolithisch ausgebildet, wobei die expandierbaren Umfangswandungen 10 bzw. Gitterstrukturen 21 durch Strukturierung eines einzelnen Ausgangsdrahts hergestellt sind.

Das erfindungsgemäße Implantat bzw. die erfindungsgemäße Vorrichtung sind vorzugsweise über einen Katheter in den menschlichen Körper einführbar, dessen Innendurchmesser höchstens 0,7 mm, insbesondere höchstens 0,51 mm, insbesondere höchstens 0,42 mm, beträgt.

Die Erfindung ist auch auf medizinische Vorrichtungen anwendbar, die temporär in einem Körperhohlraum eingesetzt werden. Insbesondere ist die Erfindung auch auf Thrombektomiedevices oder Blutfilter anwendbar, die einen expandierbaren Abschnitt aufweisen. Der expandierbare Abschnitt ist ähnlich zu der hier detailliert beschriebenen Implantatstruktur ausgebildet und fest, insbesondere monolithisch, mit einem Transportelement, beispielweise einem Führungsdraht, verbunden. Der expandierbare Abschnitt bildet vorzugsweise einen distalen Endabschnitt des Transportelements. Das Transportelement kann als Volldraht oder als Hohldraht ausgebildet sein. Insgesamt kann die gesamte medizinische Vorrichtung monolithisch aus einem Voll- oder Hohldraht gebildet sein. Der Voll- oder Hohldraht kann aus einem Verbundmaterial bestehen, das wenigstens zwei unterschiedliche Materialkomponenten enthält. Insbesondere kann ein Kerndraht mit einer Umhüllung vorgesehen sein, wobei der Kerndraht eine erste Materialkomponente und die Umhüllung eine zweite Materialkomponente enthält. Die erste Materialkomponente kann eine höhere Röntgensichtbarkeit und/oder elektrische Leitfähigkeit als die zweite Materialkomponente aufweisen.

Die Gitterstruktur 21 weist Stege 11 und Zellen 25 auf, wobei die Stege 11 die Zellen 25 vollständig begrenzen. Die Anzahl der Zellen 25 in Umfangsrichtung der Gitterstruktur 21 kann unterschiedlich sein. Insbesondere kann die Zellenanzahl je nach Verwendungszweck des Implanats bzw. der Vorrichtung eingestellt werden.

Bei der Verwendung der Gitterstruktur 21 als Stent kann die Anzahl der Zellen 25 in Umfangsrichtung zwischen 3 und 12, insbesondere zwischen 4 und 9, vorzugsweise 6, betragen. Eine derartige Zellenanzahl eignet sich für die Protektion von intrakraniellen Aneurysmen.

Bei der Verwendung der Gitterstruktur 21 als Flow Diverter, vorzugsweise ebenfalls für die Behandlung intrakranieller Aneurysmen, kann die Anzahl der Zellen 25 in Umfangsrichtung zwischen 6 und 24, insbesondere zwischen 9 und 18, vorzugsweise 12, betragen.

Für den Einsatz der Gitterstruktur 21 als Blutfilter oder Elektrode hat sich eine Zellenanzahl zwischen 4 und 16, insbesondere zwischen 6 und 12, vorzugsweise 9, als vorteilhaft erwiesen. Für denselben Einsatzzweck kann auch eine Umfangswandung 10 vorgesehen sein, die längsaxial und/oder helixförmig sich um die Längsachse der Umfangswandung 10 erstreckende Stege 11 aufweist. Dabei bildet die Umfangswandung 10 vorzugsweise zwischen 4 und 24, insbesondere zwischen 6 und 18, insbesondere zwischen 9 und 12, Zellen in Umfangsrichtung aus.

### Bezugszeichenliste

- 10: Umfangswandung
- 11: Steg
- 12: Außenfläche
- 13: Zylindermantelfläche
- 14: Erste Seitenfläche
- 15: Zweite Seitenfläche
- 16: Innenkante
- 17: Durchgangskanal
- 18: Längsschlitze
- 19: distaler Endabschnitt
- 20: Transportelement
- 21: Gitterstruktur
- 22: erste Materialkomponente
- 23: zweite Materialkomponente
- 24: Innenfläche
- 25: Zelle
- 26: Verbindungsstelle
- ID-1: Innendurchmesser vor dem Polieren
- ID-2: Innendurchmesser nach dem Polieren
- OD-3: Außendurchmesser im vollständig komprimierten Zustand
- OD-1: Außendurchmesser vor dem Polieren
- OD-2: Außendurchmesser nach dem Polieren
- ID-3: Innendurchmesser im vollständig komprimierten Zustand
- S-ID: Stegbreite am Innendurchmesser
- S-OD: Stegbreite am Außendurchmesser
- W: Wandstärke
- L: Laserstrahl
- r: Schnitttiefe
- a: Schichtdicke der ersten Materialkomponente 22
- p: Stegbreite im Übergangsbereich zwischen den Materialkomponenten 22, 23

## Patentansprüche

1. Medizinisches Implantat oder medizinische Vorrichtung mit einer radial komprimierbaren und radial expandierbaren kreiszylinderförmigen Umfangswandung (10), die einstückig aus Stegen (11) gebildet ist, wobei die Stege (11) eine im Wesentlichen kuchenstückartige Querschnittsform und jeweils zwei zur Längsachse der Umfangswandung (10) konvergierende Seitenflächen (14, 15) aufweisen, die sich parallel zu einem Radius der Umfangswandung (10) erstrecken, wobei die Stege (11) jeweils eine gekrümmte Außenfläche (12) aufweisen, die im komprimierten Zustand der Umfangswandung (10) eine äußere Zylindermantelfläche (13) der Umfangswandung (10) bildet, und wobei die Stege (11) jeweils ein Verbundmaterial aufweisen, das aus wenigstens zwei Materialkomponenten mit unterschiedlicher Röntgensichtbarkeit und/oder unterschiedlicher elektrischer Leitfähigkeit gebildet ist
**dadurch gekennzeichnet, dass**
die Stege (11) jeweils eine Innenkante (16) aufweisen, die die Seitenflächen (14, 15) verbindet, radial innerhalb der äußeren Zylindermantelfläche (13) angeordnet ist und eine Spitze der kuchenstückartigen Querschnittsform der Stege (11) bildet.

2. Medizinisches Implantat oder Vorrichtung nach einem der vorhergehenden Ansprüche,
**dadurch gekennzeichnet, dass**
die Stege (11), insbesondere die Innenkanten (16), der Umfangswandung (10) einen kreiszylinderförmigen Durchgangskanal (17) begrenzen, wobei das Verhältnis zwischen einem Innendurchmesser ID-2 des Durchgangskanals (17) im komprimierten Zustand der Umfangswandung (10) und einem Außendurchmesser OD-2 der Umfangswandung (10), insbesondere der Zylindermantelfläche (13), im komprimierten Zustand der Umfangswandung (10) höchstens 1/10, insbesondere höchstens 1/20, insbesondere höchstens 1/2, insbesondere höchstens 1/7, und/oder einem Außendurchmesser OD EXP der Umfangswandung (10), insbesondere der Zylindermantelfläche (13), im expandierten Zustand der Umfangswandung (10) höchstens 1/100, insbesondere höchstens 1/80, insbesondere höchstens 1/60, insbesondere höchstens 1/40, beträgt.

3. Medizinisches Implantat oder Vorrichtung nach einem der vorhergehenden Ansprüche,
**dadurch gekennzeichnet, dass**
die Stege (11) eine Stegdicke aufweisen, die höchstens 0,15 mm, insbesondere höchstens 0,1 mm, insbesondere höchstens 0,075 mm, insbesondere höchstens 0,06, insbesondere höchstens 0,05 mm, insbesondere höchstens 0,03 mm, beträgt.

4. Medizinisches Implantat oder Vorrichtung nach einem der vorhergehenden Ansprüche,
**dadurch gekennzeichnet, dass**
alle Stege (11) der Umfangswandung (10), die in Umfangsrichtung und/oder Längsrichtung benachbart zueinander angeordnet sind, gleichartig ausgebildet sind.

5. Behandlungssystem mit einem Implantat nach einem der vorhergehenden Ansprüche und einer Zuführeinrichtung zur Zufuhr des Implantats in ein Körperhohlorgan, insbesondere ein Blutgefäß.

6. Verfahren zur Herstellung eines medizinischen Implantats oder einer medizinischen Vorrichtung mit einer radial komprimierbaren und radial expandierbaren kreiszylinderförmigen Umfangswandung (10), die einstückig aus Stegen (11) gebildet ist, wobei die Stege (11) eine im Wesentlichen kuchenstückartige Querschnittsform und jeweils zwei zur Längsachse der Umfangswandung (10) konvergierende Seitenflächen (14, 15) aufweisen, die sich parallel zu einem Radius der Umfangswandung (10) erstrecken, wobei das medizinische Implantat oder die medizinische Vorrichtung aus einem kreiszylinderförmigen Vollmaterial hergestellt wird, wobei das Vollmaterial ein Verbundmaterial ist, das in wenigstens einem Längsabschnitt mit einem Schneidwerkzeug radial vollständig durchtrennt wird derart, dass die radial komprimierbare und radial expandierbare Umfangswandung (10) aus Stegen (11) mit einer kuchenstückartigen Querschnittsform gebildet wird, wobei das Verbundmaterial aus wenigstens zwei Materialkomponenten mit unterschiedlicher Röntgensichtbarkeit und/oder unterschiedlicher elektrischer Leitfähigkeit gebildet ist.

7. Verfahren nach Anspruch 6,
**dadurch gekennzeichnet, dass**
mit dem Schneidwerkzeug Längsschlitze (18) gebildet werden, die das Vollmaterial radial vollständig durchdringen, wobei die Längsschlitze (18) eine Länge aufweisen, die kleiner als eine Gesamtlänge des Vollmaterials ist.

8. Verfahren nach Anspruch 7,
**dadurch gekennzeichnet, dass**
entlang einer Längsachse des Vollmaterials mehrere Längsschlitze (18) ausgeformt werden, die in Umfangsrichtung versetzt zueinander angeordnet sind.

9. Verfahren nach Anspruch 7 oder 8,
**dadurch gekennzeichnet, dass**
die Längsschlitze (18) in Umfangsrichtung in gleichmäßigen Abständen zueinander in das Vollmaterial eingebracht werden.

10. Verfahren nach einem der Ansprüche 6 bis 9,
**dadurch gekennzeichnet, dass**
das Schneidwerkzeug ein Laserstrahl L ist, insbesondere wobei der Laserstrahl L eine parallele oder zum Zentrum des Vollmaterials sich aufweitende Strahlführung aufweist.

11. Verfahren nach einem der Ansprüche 6 bis 10,
**dadurch gekennzeichnet, dass**
die Stege (11) der Umfangswandung (10) anschließend, insbesondere nach der Bearbeitung mit dem Schneidwerkzeug, poliert werden.

## Claims

1. A medical implant or medical device having a radially compressible and radially expandable, cylindrical, peripheral wall (10) formed by webs (11) in one piece, the webs (11) having a cross section shaped essentially like a piece of cake and having two side faces (14, 15) converging toward the longitudinal axis of the peripheral wall (10), extending parallel to a radius of the peripheral wall (10), the webs (11) each having a curved outer surface (12) forming an outer lateral cylindrical surface (13) of the peripheral wall in the compressed state of the peripheral wall (10), and the webs (11) comprising of a composite material formed by at least two material components having different radiopacities and/or different electrical conductivities,
**characterized in that**
the webs (11) each have an inner edge (16) connecting the side faces (14, 15), arranged on the inside radially of the outer lateral cylindrical surface (13) and forming a tip of the cross section of the webs (11) shaped like a piece of cake.

2. The medical implant or device according to any one of the preceding claims,
**characterized in that**
the webs (11), in particular the inner edges (16) of the peripheral wall (10) delineate a cylindrical through-channel (17), wherein the ratio between an inside diameter ID-2 of the through-channel (17) of the peripheral wall (10) in the compressed state and an outside diameter OD-2 of the peripheral wall (10), in particular of the lateral cylindrical surface (13) in the compressed state of the peripheral wall (10), amounts to at most 1/10, in particular at most 1/20, in particular at most 1/2, in particular at most 1/7, and/or an outside diameter OD EXP of the peripheral wall (10), in particular of the lateral cylindrical surface (13) in the expanded state of the peripheral wall (10) amounts to at most 1/100, in particular at most 1/80, in particular at most 1/60, in particular at most 1/40.

3. The medical implant or device according to any one of the preceding claims,
**characterized in that**
the webs (11) have a web thickness amounting to at most 0.15 mm, in particular at most 0.1 mm, in particular at most 0.075 mm, in particular at most 0.06 mm, in particular at most 0.05 mm, in particular at most 0.03 mm.

4. The medical implant or device according to any one of the preceding claims,
**characterized in that**
all webs (11) of the peripheral wall (10) arranged next to one another in the peripheral direction and/or in the longitudinal direction are designed to be identical.

5. The treatment system with an implant according to any one of the preceding claims and a feed device for feeding the implant into a hollow organ in the body, in particular a blood vessel.

6. The method for producing a medical implant or a medical device having a radially compressible and radially expandable cylindrical peripheral wall (10) formed from webs (11) in one piece, wherein the webs (11) have a cross section shaped essentially like a piece of cake, and having two side faces (14, 15) converging toward the longitudinal axis of the peripheral wall (10), extending parallel to a radius of the peripheral wall (10), wherein the medical implant or the medical device is produced from a cylindrical solid material, wherein the solid material is a composite material severed completely radially by a cutting tool in at least one longitudinal section, such that the radially compressible and radially expandable peripheral wall (10) is formed by webs (11) with a cross section in the form of a piece of cake, wherein the composite material is formed from at least two material components with different radiopacities and/or different electrical conductivities.

7. The method according to claim 6,
**characterized in that**
longitudinal slots (18) permeating radially completely through the solid material are formed by the cutting tool, wherein the longitudinal slots (18) have a length smaller than the total length of the solid material.

8. The method according to claim 7,
**characterized in that**
multiple longitudinal slots (18) arranged offset from one another in the peripheral direction are formed along a longitudinal axis of the solid material.

9. The method according to claim 7 or 8,
**characterized in that**
the longitudinal slots (18) are formed in the solid material at uniform intervals from one another in the peripheral direction.

10. The method according to any one of claims 6 to 9,
**characterized in that**
the cutting tool is a laser beam L, in particular wherein the laser beam L has a beam guidance that is parallel or becomes wider relative to the center of the solid material.

11. The method according to any one of claims 6 to 10,
**characterized in that**
the webs (11) are connected to the peripheral wall (10), in particular being polished after processing them with the cutting tool.

## Revendications

1. Implant médical ou dispositif médical, comportant une paroi périphérique (10) de forme cylindrique compressible radialement et expansible radialement et qui est réalisé en une pièce à partir de traverses (11), les traverses présentant une forme de section transversale (11) ressemblant sensiblement à une part de gâteau et deux surface latérale (14, 15) convergent respectivement vers l'axe longitudinal de la paroi périphérique (10) et qui s'étendent parallèlement à un rayon de la paroi périphérique (10), les traverses (11) présentant respectivement une surface extérieure courbée (12) qui, à l'État comprimé de la paroi périphérique (10), constitue une surface de chemise cylindrique (13) de la paroi périphérique (10) et les traverses (11) présentant respectivement un matériau composite qui est constitué d'au moins deux composants de matériaux à visibilités radiographiques différentes et/ou à conductivité électrique différente,
**caractérisée en ce que**
les traverses (11) présentent respectivement un bord intérieur (16) qui relie les surfaces latérales (14, 15), et disposer radialement à l'intérieur de la surface de chemise cylindrique extérieure (13) et constitue une pointe de la forme de section transversale en forme de parts de gâteau des traverses (11) .

2. Implant ou dispositif médical selon une des revendications précédentes,
**caractérisé en ce que**
les traverses (11), en particulier les bords intérieurs (16), de la paroi périphérique (10) limitent un canal traversant de forme cylindrique (17), le rapport entre un diamètre intérieur ID-2 du canal traversant (17) à l'état comprimé de la paroi périphérique (10) et un diamètre extérieur OD-2 de la paroi périphérique (10), en particulier de la surface de chemise cylindrique (13), à l'état comprimé de la paroi périphérique (10) s'élève à au plus 1/10, en particulier au plus 1/20, en particulier au plus 1/2, en particulier au plus 1/7, et/ou un diamètre extérieur OD EXP de la paroi périphérique (10), en particulier de la surface de chemise cylindrique (13), à l'état dilaté de la paroi périphérique (10) au plus 1/100, en particulier au plus 1/80, en particulier au plus 1/60, en particulier au plus 1/40.

3. Implant ou dispositif médical selon une des revendications précédentes,
**caractérisé en ce que**
les traverses (11) présentent une épaisseur de traverses qui s'élève à au plus 0,15 mm, en particulier au plus 0,1 mm, en particulier au plus 0,075 mm, en particulier au plus 0,06, en particulier au plus 0,05 mm, en particulier au plus 0,03 mm.

4. Implant ou dispositif médical selon une des revendications précédentes,
**caractérisé en ce que**
toutes les traverses (11) de la paroi périphérique (10) qui sont disposées à proximité les unes des autres dans le sens référentiel et/ou le sens longitudinal ont une conformation similaire.

5. Systèmes de traitement avec un implant selon une des revendications précédentes et un dispositif d'acheminement pour l'acheminement de l'implant dans un organe corporel creux, en particulier un vaisseau sanguin.

6. Procédé de fabrication d'un implant médical ou d'un dispositif médical comportant une paroi périphérique (10) compressible radialement et expansible radialement qui est réalisée en une pièce à partir de traverses (11), les traverses (11) présentant une forme de section sensiblement en forme de part de gâteau et deux surfaces latérales (14, 15) convergeant respectivement vers l'axe longitudinal de la paroi périphérique (10) et qui s'étendent parallèlement un rayon de la paroi périphérique (10), l'implant médical ou le dispositif médical étant fabriqué à partir d'un matériau plein de forme cylindrique, le matériau plein étant un matériau composite qui est coupé intégralement radialement avec un outil de coupe de manière à ce que la paroi périphérique (10) compressible radialement et expansible radialement soit composée de traverses (11) ayant une forme de section transversale en forme de part de gâteau, le matériau composite étant composé d'au moins deux composants de matériau à visibilité radiographique différente et/ou conductivité électrique différente.

7. Procédé selon la revendication 6,
**caractérisé en ce**
**qu'**on réalise avec l'outil de coupe des fentes longitudinale (18) qui traversent totalement le matériau plein radialement, les fentes longitudinales (18) présentant une longueur qui est inférieure à une longueur totale du matériau plein.

8. Procédé selon la revendication 7,
**caractérisé en ce que**,
le long d'un axe longitudinal du matériau plein, sont pratiquées plusieurs fentes longitudinales (18) qui sont disposées décalées les unes des autres dans le sens circonférentiel.

9. Procédé selon la revendication 7 ou 8,
**caractérisé en ce que**
les fentes longitudinales (18) sont pratiquées dans le sens circonférentiel à distance régulière les unes des autres dans le matériau plein.

10. Procédé selon une des revendications 6 bis 9,
**caractérisé en ce que**
l'outil de coupe est un rayon laser L, le rayon laser L présentant en particulier un guide de rayon parallèle ou s'élargissant en direction du centre du matériau plein.

11. Procédé selon une des revendications 6 bis 10,
**caractérisé en ce que**
les traverses (11) de la paroi périphérique (10) sont ensuite polies, en particulier après usinage avec l'outil de coupe.
